(19) Europäisches Patentamt / European Patent Office / Office européen des brevets

(11) **EP 1 974 240 B1**

(12) **EUROPEAN PATENT SPECIFICATION**

(45) Date of publication and mention
of the grant of the patent:
**18.12.2013 Bulletin 2013/51**

(51) Int Cl.:
*H04N 5/225* (2006.01)     *A61B 1/05* (2006.01)

(21) Application number: **06849994.6**

(22) Date of filing: **19.12.2006**

(86) International application number:
**PCT/US2006/048752**

(87) International publication number:
**WO 2007/126429 (08.11.2007 Gazette 2007/45)**

(54) **IN VIVO SENSOR WITH PANORAMIC CAMERA**

IN-VIVO-SENSOR MIT PANORAMAKAMERA

CAPTEUR IN VIVO AVEC CAMÉRA PANORAMIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.01.2006 US 760079 P**
**19.01.2006 US 760794 P**

(43) Date of publication of application:
**01.10.2008 Bulletin 2008/40**

(73) Proprietor: **Capso Vision, Inc.**
**Saratoga, CA 95070 (US)**

(72) Inventors:
• **WANG, Kang-huai**
**Saratoga, CA 95070 (US)**
• **WILSON, Gordon**
**Saratoga, CA 95070 (US)**

(74) Representative: **Betten & Resch**
**Theatinerstrasse 8**
**80333 München (DE)**

(56) References cited:
WO-A1-95/02841          WO-A2-2004/032621
WO-A2-2004/096008       US-A- 5 473 474
US-A1- 2003 171 653     US-A1- 2005 004 474
US-A1- 2005 049 462     US-A1- 2005 146 644
US-B2- 6 748 797

• Yagi, Yasushi: "Omnidirectional Sensing and its Applications" IEICE TRANSACTIONS on Information and Systems vol. E82-D, no. 3, 20 March 1999 (1999-03-20), pages 568-579, XP002589983 ISSN: 0916-8532 Retrieved from the Internet: URL:http://citeseerx.ist.psu.edu/viewdoc/d ownload? doi=10.1.1.29.8128&rep=rep1&type=p df [retrieved on 2010-07-01]
• LEHNER D L ET AL: "Characterization of the panoramic annular lens", EXPERIMENTAL MECHANICS, SPRINGER NEW YORK LLC, US LNKD- DOI:10.1007/BF02328575, vol. 36, no. 4, 1 December 1996 (1996-12-01), pages 333-338, XP009138656, ISSN: 0014-4851

## Description

## Background

various autonomous devices have been developed that are configured to capture an image from within *in vivo* passages and cavities within a body, such as those passages and cavities within the gastrointestinal (GI) tract. These devices typically comprise a digital camera housed within a capsule along with light sources for illumination. The capsule may be powered by batteries or by inductive power transfer from outside the body. The capsule may also contain memory for storing captured images and/or a radio transmitted for transmitting data to an *ex vivo* receiver outside the body.

A common diagnostic procedure involves the patient swallowing the capsule, whereupon the camera begins capturing images and continues to do so at intervals as the capsule moves passively through the cavities made up of the inside tissue walls of the GI tract under the action of peristalsis. The capsule's value as la diagnostic tool depends on it capturing images of the entire interior surface of the organ or organs of interest. Unlike endoscopes, which are mechanically manipulated by a physician, the orientation and movement of the capsule camera are not under an operator's control and are solely determined by the physical characteristics of the capsule, such as its size, shape, weight, and surface roughness, and the physical characteristics and actions of the bodily cavity. Both the physical characteristics of the capsule and the design and operation of the imaging system within it must be optimized to minimize the risk that some regions of the target lumen are not imaged as the capsule passes through the cavity.

Two general image-capture scenarios may be envisioned, depending on the size of the organ imaged. In relatively constricted passages, such as the esophagus and the small intestine, a capsule which is oblong and of length less than the diameter passage, will naturally align itself longitudinally within the passage. Typically, the camera is situated under a transparent dome at one (or both) ends of the capsule. The camera faces down the passage so that the center of the image comprises a dark hole. The field of interest is the intestinal wall at the periphery of the image

The capsule 100 is encased in a housing 101 so that it can travel in vivo inside an organ 102, such as an esophagus or a small intestine, within an interior cavity 104. The capsule may be in contact with the inner surfaces 106,108 of the organ, and the camera lens opening110 can capture images within its field of view 112. The capsule may include an output port 114 for outputting image data, a power supply 116 for powering components of the camera, a memory 118 for storing images, image compression 120 circuitry for compressing images to be stored in memory, an image processor 122 for processing image data, and LEDs 126 for illuminating the surfaces 106,108 so that images can be captured from the light that is scattered off of the surfaces.

It is desirable for each image to have proportionally more of its area to be intestinal wall and proportionally less the receding hole in the middle. Thus, a large FOV is desirable. A typical FOV is 140°. Unfortunately, a simple wide-angle lens will exhibit increased distortion and reduced resolution and numerical aperture at large field angles. High-performance wide-angle and "fisheye" lenses are typically large relative to the aperture and focal length and consist of many lens elements. A capsule camera is constrained to be compact and low-cost, and these types of configurations are not cost effective. Further, these conventional devices waste illumination at the frontal area of these lenses, and thus the power use to provide such illumination is also wasted. Since power consumption is always a concern, such wasted illumination is a problein. Still further, since the intestinal wall within the filed of view extends away from the capsule, it is both foreshortened and also requires considerable depth of field to image clearly in its entirety. Depth of field comes at the expense of exposure sensitivity.

The second scenario occurs when the capsule is in a cavity, such as the colon, whose diameter is larger than any dimension of the capsule. In this scenario the capsule orientation is much less predictable, unless some mechanism stabilizes it. Assuming that the organ is empty of food, feces, and fluids, the primary forces acting on the capsule are gravity, surface tension, friction, and the force of the cavity wall pressing against the capsule. The cavity applies pressure to the capsule, both as a passive reaction to other forces such as gravity pushing the capsule against it and as the periodic active pressure of peristalsis. These forces determine the dynamics of the capsule's movement and its orientation during periods of stasis. The magnitude and direction of each of these forces is influenced by the physical characteristics of the capsule and the cavity. For example, the greater the mass of the capsule, the greater the force of gravity will be, and the smoother the capsule, the less the force of friction. Undulations in the wall of the colon will tend to tip the capsule such that the longitudinal axis of the capsule is not parallel to the longitudinal axis of the colon.

Also, whether in a large or small cavity, it is well known that there are sacculations that are difficult to see from a capsule that only sees in a forward looking orientation. For example, ridges exist on the walls of the small and large intestine and also other organs. These ridges extend somewhat perpendicular to the walls of the organ and are difficult to see behind. A side or reverse angle is required in order to view the tissue surface properly. Conventional devices are not able to see such surfaces, since their FOV is substantially forward looking. It is important for a physician to see all areas of these organs, as polyps or other irregularities need to be thoroughly observe for an accurate diagnosis. Since conventional capsules are unable to see the hidden areas around the ridges, irregularities may be missed, and critical diagnoses of serious medical conditions may be flawed. Thus, there exists a need for more accurate viewing of these often missed areas with a capsule.

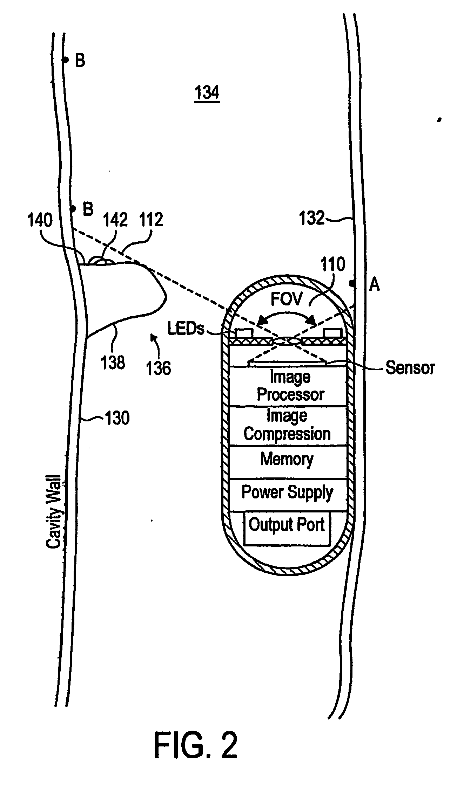

Figure 2 shows a relatively straightforward example where the passage 134, such as a human colon, is relatively horizontal, with the exception of the ridge 136, and the capsule sits on its bottom surface 132 with the optical axis of the camera parallel to the colon longitudinal axis. The ridge illustrates a problematic viewing area as discussed above, where the front surface 138 is visible and observable by the capsule 100 as it approaches the ridge. The backside of the capsule 140, however, is not visible by the capsule lens, as the limited FOV 110 does not pick up that surface. Specifically, the range 110 of the FOV misses part of the surface, and moreover misses the irregularity illustrated as polyp 142.

Three object points within the field of view 110 are labeled A, B, and C. The object distance is quite different for these three points, where the range of the view 112 is broader on one side of the capsule than the other, so that a large depth of field is required to produce adequate focus for all three simultaneously. Also, if the LED (light emitting diode) illuminators provide uniform flux across the angular FOV, then point A will be more brightly illuminated than point B and point B more than point C. Thus, an optimal exposure for point B results in over exposure at point A and under exposure at point C. For each image, only a relatively small percentage of the FOV will have proper focus and exposure, making the system inefficient. Power is expended on every portion of the image by the flash and by the imager, which might be an array of CMOS or CCD pixels. , Moreover, without image compression, further system resources will be expended to store or transmit portions of images with low information content. In order to maximize the likelihood that all surfaces within the colon are adequately imaged, a significant redundancy, that is, multiple overlapping images, is required.

One approach to alleviating these problems is to reduce the instantaneous FOV but make the FOV changeable. Patent application 2005/0146644 discloses an *in-vivo* sensor with a rotating field of view. The illumination source may also rotate with the field of view so that regions outside the instantaneous FOV are not wastefully illuminated. This does not completely obviate the problem of wasteful illumination, and furthermore creates other power demands when rotating. Also, this innovation by itself does not solve the depth of field and exposure control problems discussed above.

Thus there exists a need in the art for a more improved system and method for in-vivo viewing of organs with a capsule, where better viewing angles can provide better observation of tissue surfaces. As will be seen below, the invention provides such a system and a method that overcomes the problems of the prior art, and they do so in an elegant manner.

Prior art document WO 2004/096008 A2 provides an in vivo imaging device with a curved reflective element and for example a concave portion of an outer shell of such device. Such curved reflective element may for example reflect onto an image sensor light rays from an object where such light rays before reflection are substantially parallel to the plane of the image sensor. With regard to the concave portion of the outer shell, two different window sections for incoming and outgoing light rays are arranged with an angle to each other.

WO 95/02841 A1 discloses a panoramic imaging system for projecting a 360 degree cylindrical field of view onto a two-dimensional annular format has a panoramic imaging block with a concentric axis of symmetry, two refractive surfaces and two reflective surfaces. The first reflective surface is a concave conicoid of revolution with the conic constant in the range from -0.6 to +2.0. In an embodiment of the invention, the second refractive surface (the last in the path of rays) is flat, while the first reflective surface, the second reflective surface and the first refractive surface are all spherical.

The publication "Characterization of the panoramic annular lens" by Lehner, D. L et al., Experimental Mechanics, Springer New York LLC, vol. 36, no. 4, 1 December 1996, pages 333-338, describes a panoramic annular lens consisting of a single piece of glass with spherical surfaces that produces a flat annular image of the entire 360° surround of the optical axis of the lens. Moreover, the lens is adapted to map elements from object to image space via a constant aspect ratio polar mapping.

US 2003/0171653 A1 provides a capsule endoscope with lighting means for illuminating the interior of a living body, an image pickup means for capturing an image of a site illuminated by the lighting means, and a transparent cover which houses the image pickup means and the lighting means within a sealed, cylindrical-shaped capsule case. The image pickup means includes an objective optical system having a depth of field. An object within the depth of field of the objective optical system Is illuminated more evenly than possible with a single lighting means by arranging multiple lighting means at the periphery of the objective optical system and causing the beams from individual lighting means to overlap in specified ways so that a nearly uniform illumination of an object of interest is achieved. Light diffusers may be employed to diffuse the light from the light sources, and a color correction filter may be used.

**Brief Description of the Drawings**

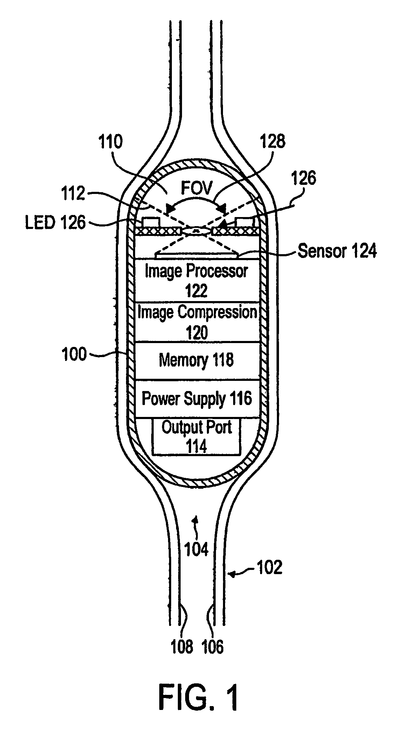

Figure 1 illustrates the field of view of a capsule camera in the small intestine.

Figure 2 illustrates the field of view of a capsule camera in the large intestine.

Figs. 3a-b illustrates the panoramic annular lens (PAL) in an endoscopic capsule.

Figure 4 illustrates a capsule with the optical axis of the lens oriented along the longitudinal axis of a cylinder.

Figure 5 illustrates a geometry for a panoramic camera where the LEDs lie below and to the outside of the first reflector.

Figure 6 illustrates the ray paths on entering a capsule.

Figure 7 illustrates another embodiment of panoramic capsule camera and LED illuminators where the region between the LED, the housing, and the outside of the first reflector is filled with an index-matching potting material.

Figure 8 further illustrates the ray paths for the embodiment of Figure 7.

## Summary

We present a capsule camera that utilizes a panoramic camera. Panoramic cameras provide a FOV that exceeds 180° but with an obscuration at the center of the FOV. For example the FOV might be a full 360° of latitude with angles of longitude ranging from 160° to 200°. Panoramic annular lenses are first described in US 4,566,763, and refinements are described in US 5,473,474. The panoramic FOV reduces the possibility that the camera will fail to image some portion of the organ as it passes through it, even if its orientation is variable. We also disclose the use of a balloon or balloons attached to the capsule that position the capsule near the center of a cavity, such as the colon, whose diameter is larger than that of the capsule, and maintain a longitudinal capsule orientation. The walls of the colon within the panoramic FOV are then nearly equidistant from the camera, reducing the required depth of field and simplifying exposure.

## Detailed description of the invention

The invention is directed to a panoramic in vivo camera system. In one embodiment, the.system is configured in a capsule, and a panoramic camera is encased within the capsule and configured to capture a panoramic view of tissue surface around or otherwise about the capsule. An optical system is configured to illuminate an area of interest outside the capsule to the camera system. More generally, the invention provides a remote image capturing system that includes a housing that acts as a delivery system such as a capsule for example. This housing encloses the remote image capturing system for function and protection. A camera is enclosed within the housing and is configured to capture a panoramic image of an environment immediately surrounding the housing, such as within a subject's esophagus, large or small intestine, or other organ. A light source is also.included to provide an illumination source for the camera. At least one power source can be electronically coupled with the light source and the processing system to provide power to each.

The optical system may include a transparent window for exposing an image onto the panoramic camera. The panoramic camera can be configured with a longitudinal ' field of view and a latitudinal field of view, where that the camera is configured to capture a panoramic image of an organ.

In one embodiment, the invention is directed to an in vivo image capturing system, where the system includes a camera having an entrance pupil, the camera configured to capture a panoramic image of an environment surrounding the camera, wherein the panoramic image is captured on a single image plane. The system further includes a light source for providing an illumination source for the camera and a closed housing for enclosing the camera and the light source. The system may further comprise a power source for providing power to the light source and the camera, and the power source may be a battery located within the capsule. The power source may be an inductive power source located outside the capsule and configured to induce power into the capsule to power the camera and illumination source. The closed housing may include a transparent window for passing light into the panoramic camera, wherein the panoramic camera is configured with a longitudinal field of view defined by a range of view angles relative to a longitudinal axis of the capsule and a latitudinal field of view defined by a panoramic range of azimuth angles about the longitudinal axis such that the camera can capture 8, panoramic image covering substantially a 360 deg latitudinal FOV and a longitudinal FOV ranging from some angle greater than 0deg to another angle greater than 90deg.

The camera may have a longitudinal axis such that the angular FOV includes a plane substantially in its entirety, wherein the plane is perpendicular to the axis and intersects the pupil. The system may include an optical system that has a panoramic annular lens configured to focus an image onto the single image plane. The capsule may be configured with a body of oblong shape and having a longitudinal axis along the length of the body, the system further including an optical system having a panoramic annular lens that enables images to be captured by the panoramic camera radially about the longitudinal axis and onto the single image plane. The camera may be oriented within the capsule to capture images radially about the longitudinal axis and has a FOV that is substantially perpendicular with respect to the capsule to enable viewing of a tissue surface oriented parallel to the principle direction of travel of the capsule and to enable capturing an image of the tissue surface onto the single image plane. The capsule may have a housing that is oblong in shape, and wherein the camera is oriented within the housing to capture images radially about the longitudinal axis and along the axis of the body, the system further including an optical system having a panoramic annular lens and a window located substantially surrounding area of the oblong body for receiving light rays from an object into the panoramic annular lens. The longitudinal axis may be a concentric axis of symmetry for the panoramic annular lens.

The panoramic annular lens may have a concentric axis of symmetry and includes two refractive surfaces and two reflective surfaces configured to allow incoming light to pass through a first refractive surface into a transparent medium. Alternatively, the panoramic annular lens may have a concentric axis of symmetry and includes two refractive surfaces and two reflective surfaces configured to allow incoming light to pass through a first refractive surface into a transparent medium, to be reflected by a first reflective surface, then reflected by a second reflective surface, and then to exit the medium through a second refractive surface. The panoramic annular lens may be configured to produce an image with a cylindrical field of view from a point-of view on the concentric axis. The reflective and refractive surfaces may be conicoid surfaces of revolution, and wherein the conic constant of the first reflector is in the range of -0.6 to +2. The reflective and refractive surfaces may also be conicoid surfaces of revolution, wherein the conic constant of the first reflective surface is in the range of -0.6 to +2, where the conic constant of the second reflective surface is chosen to be less than that of the first. The reflective and refractive surfaces may be one of conicoid surfaces of revolution, spheroidal surfaces, or aspheroidal surfaces:

The system may include further a data processor configured to generate a continuous image made up of multiple captured images. The data processor may be located outside the capsule. The data processor may be configured to generate a continuous image made up of multiple contiguous captured images. The system may further include a memory configured to store captured images, where the memory is an electronic memory, or other type of memory. The memory may be located within the capsule. The memory may be located within the capsule, wherein the processor is configured to compress image data and to store the compressed image data in the electronic memory.

The memory may be located outside the capsule, wherein image data is stored in the memory for subsequent processing, and the system may further comprise a remote processor configured to compress image data and to store the compressed image data in the memory.

The system may further include a transmission module configured to transmit images captured by the panoramic camera to a remote receiver configured to receive captured images. The transmission module may be configured to transmit images captured by the panoramic camera to a remote receiver having data storage configured to store captured images for processing by an image processor.

In operation, the system may perform a method of in-vivo imaging, by providing a device for panoramic in-vivo imaging of the gastrointestinal tract onto a single image plane. The method further includes emitting electromagnetic radiation from the capsule; and receiving reflections of the electromagnetic radiation from tissue surfaces for use in forcing a panoramic image of the tissues from a field of view that includes substantially all directions perpendicular to the principle direction of travel through the gastrointestinal tract.

The method may further include uploading the compressed image data to a host computer for'processing, and it may first perform compression on images detected by an image sensor to produce compressed image data. The process may further include performing compression on images detected by an image sensor in the capsule to produce compressed image data, storing the compressed image data in a memory in the capsule, then uploading the compressed image data to a host computer. The step of uploading the images to a host computer may further comprise retrieving the capsule housing and uploading the images by connepting the capsule to the host computer. Uploading the images to a host computer may further comprise uploading the images by transmitting the images from the capsule to the host computer.

In a wireless system the method may further include uploading the compressed image data to a host computer with a wireless signal. And the method may also perform compression on images detected by an image sensor to produce compressed image data, and then upload the compressed image data to a host computer via a wireless communication signal. The method may perform the process by performing compression on images detected by an image sensor in the capsule to produce compressed image data, storing the compressed image data in a memory in the capsule; and then uploading the compressed image data to a host computer via a wireless communication signal. More specifically, the method for in-vivo imaging with a camera encased within a capsule may include providing a capsule housing having a portion at least partially transparent to electromagnetic radiation in a given wavelength range, ingesting said capsule by a patient, emitting electromagnetic radiation in said wavelength range from said capsule, detecting images from reflections of said electromagnetic radiation with an image sensor using a panoramic camera, performing compression on images detected by an image sensor in said capsule before storing said images in a transistor memory in said capsule, retrieving said capsule from said patient, and then uploading said images to a host computer.

In yet another embodiment, the invention provides a capsule configured to house a camera for in-vivo imaging, where the capsule includes a panoramic camera and an illuminating light source. The papsule may be a closed capsule having a first set of inner and outer transparent-window-surface regions demarcated by the intersections of incoming image rays within the camera FOV with the interior and exterior surfaces of the window and a second set of inner and outer transparent-window-surface regions demarcated by the intersections of illuminating light rays, passing from within the capsule to the exterior, with the interior and exterior surfaces of the window; wherein the inner surface regions from each set and the outer surface regions from each set are sufficiently non-overlapping such that wherever incoming image rays and outgoing illuminating rays intersect at a common point on a window surface the angle between the outgoing ray and the surface normal exceeds the angle between the incoming ray and the surface normal such that the reflection of the outgoing ray from the surface is not within the camera FOV. The inner surface regions from each set may be non overlapping. Alternatively, the outer surface regions from each set are non overlapping. The capsule may further include a reflector configured to reflect illuminating light from the illuminating light source away from the camera and toward a subject surface. The camera may include a lens configured to receive illuminating light reflected from a subject surface, the capsule having a reflector configured to reflect illuminating light from the illuminating light source away from the camera in a manner that prevents stray light from directly entering the camera lens without being scattered by a subject surface.

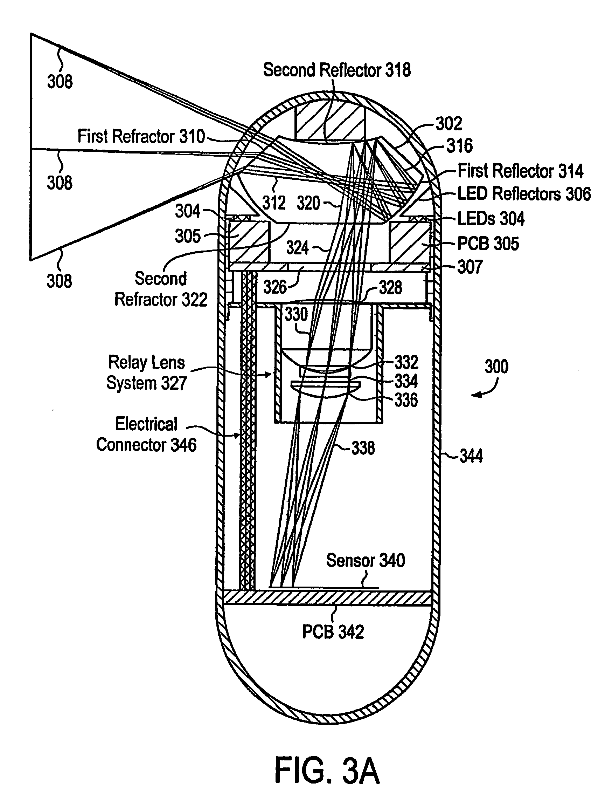

Another embodiment of a capsule camera 300 having a panoramic annular lens (PAL) 302, is shown schematically in Figure 3a. The lens 302 has a concentric axis of symmetry and comprises two refractive surfaces and two reflective surfaces such that incoming light passes through the first refractive surface into a transparent medium, is reflected by the first reflective surface, then by the second reflective surface, and then exits the medium through the second refractive surface.

The capsule camera 300 includes LED outputs 304 configured to illuminate outside the capsule onto a subject, such as tissue surface being imaged. The LEDs include LED reflectors 306 configured to reflect any stray LED light away from the lens 302. The purpose of the LED light rays is to reflect off of the tissue surface ant into the lens 302 so that an image can be recorded. The reflectors serve to reflect any light from the light source, the LEDs, away from the lens 302 so that only light rays reflected from the tissue surface will be imaged. The LEDs are connected to printed circuit boards PCBs 305 that are connected to each other via a conductor wire or plate 307, distributing power to each LED. The lens 302 is configured to receive and capture light rays 308 that are reflected off of an outside surface, such as a tissue surface, and receives the reflected rays through a first refractor 310. The defracted rays 312 are transmitted to a first reflector 314, which transmits reflected rays 316 onto the surface, of a second reflector 318. The second reflector then reflects reflected rays 320 through a second refractor 322, sending refracted rays 324 through opening 326 and into a replay lens system 327.

The system shown is a Cooke triplet relay lens, and it includes a first lens 328 for receiving the refracted rays 324 from the second refractor 322. The first lens focuses the light rays 330 onto a second lens 332. Those focused rays 334 are sent to third lens 336, which focuses rays 338 onto sensor 340. The sensor is mounted on PCB 342, which is connected to the capsule outer walls 344.

The capsule 300 further includes electrical conductor 346 connecting the PCB 342 holding the sensor to the conductor plate or wire 307. The electrical conductor 346 is configured for powering the LEDs 304 through the conductor plate 307 and PCBs 305 that hold the LEDs 304. Those skilled in the art will understand that other configurations are possible without departing from the spirit and scope of the invention, which is defined by the appended claims.

The PAL lens 302 produces an image with a cylindrical FOV from a point-of view on the concentric axis. The surfaces of the lens 302 may be conicoid surfaces of revolution, or they may be other spheroidal or aspheroidal shapes. In a preferred embodiment, the conic constant of the first reflector is optimally in the range of -0.6 to +2 with the conic constant of the second reflector chosen to be less than that of the first. For example, the first reflector might be ellipsoidal with the second reflector hyperboloidal.

A relay image system after the PAL lens 302 forms an image on a two-dimensional light sensor 340 that may be a commonly known sensor such as a CMOS or CCD array. Figure 3a illustrates a Cooke triplet relay lens 327. There exists other configurations that are well known in the art and include double-Gauss configurations.

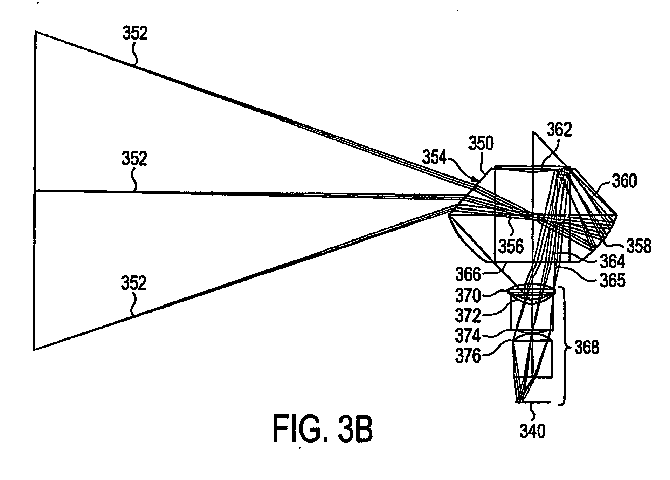

Referring to Figure 3b, a lens design is shown a lens design 350 with reduced total track length for the relay imaging system. The lens 350 is configured to receive and capture light rays 352. that are reflected off of an outside surface, such as a tissue surface, and receives the reflected rays through a first refractor 354. The refracted rays 356 are transmitted to a first reflector 358, which transmits reflected rays 360 onto the surface of a second reflector 362. The second reflector then reflects reflected rays 364 through a second refractor 366, sending refracted rays 365 through opening 326 and into a relay lends system 368. The relay lens system is configured with lenses 370,372,374,376 for focusing the light rays onto sensor 340. The reduced total track of the imaging system allows for more compact design and manufacture of a camera, inside the imaging capsule, providing for different designs and configurations.

In either embodiment illustrated in Figures 3a or 3b, the capsule camera may instead use other panoramic lens designs including those that utilize a single reflector in the shape of a paraboloid, spheroid, cone, or tetrahedron, in conjunction with relay lenses, to map a cylindrical FOV onto an image plane. Other designs may utilize a single concave ellipsoidal reflector.

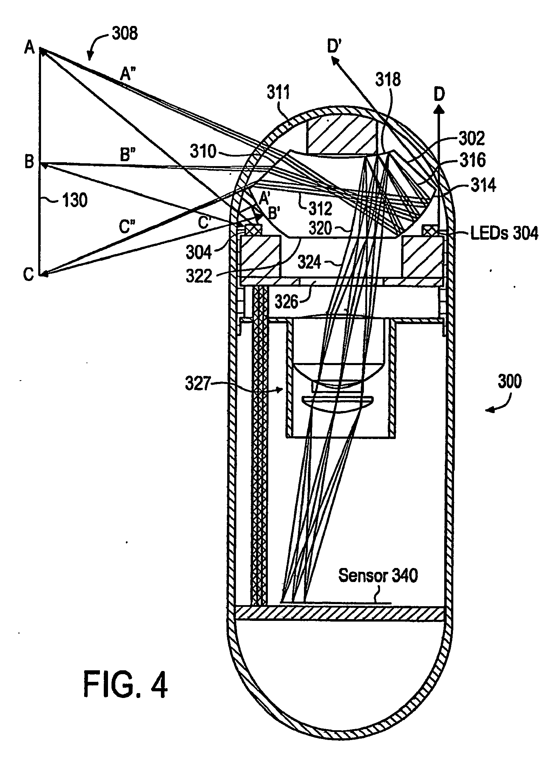

Referring again to Figure 3a, this figure illustrates the PAL lens 302 in an endoscopic capsule. The capsule is shown as a cylinder with hemispherical ends. In a preferred embodiment, at least a circumferential band of the capsule housing must be transparent to allow illuminating light to exit the capsule and to allow image light to enter the capsule. In a preferred embodiment, the optical axis of the lens is oriented along the longitudinal axis of a cylinder (Figure 4) or along the major axis of an ellipsoid in an ellipsoid capsule configuration.

Referring to Figure 4, the LED 304 is configured to emit light rays A,B,C on a subject surface 130, such as tissue surface. The capsule wall 311 would be transparent to the incoming rays A",B",C", which are each received through the first refractor 310. The LED light rays A',B',C' are reflected off of the lens 302, so that the LED rays do not distort the image captured by the sensor 340.

Just as discussed above, the lens 302 is configured to receive and capture light rays 308 that are reflected off of an outside surface, such as a tissue surface, and receives the reflected rays through a first refractor 310. The refracted rays 312 are transmitted to a first reflector 314, which transmits reflected rays 316 onto the surface of a second reflector 318. The second reflector then reflects reflected rays 320 through a second refractor 322, sending refracted rays 324 through opening 326 and into a relay lens system 327.

The capsule might have any oblong shape or a spherical shape, however, and the optical axis may have any orientation within the capsule. Any electrical connections made from the end of the lens above the 2nd reflector to the region below the 1st reflector will obscure the field of view to some extent. The sensor is necessarily below the lens. Thus, electronics placed above the lens and the sensor cannot share a common power supply and control circuitry without obscuring connecting wires. Therefore, it is advantageous to place the lens with the second reflector adjacent to one end of the capsule. The quasi-spherical shape of the PAL fits economically within the hemispherical end of the capsule, as well. However, if a balloon is attached to the end of the capsule above the lens, it may be desirable to lower the lens so that the balloon is not in the FOV. If batteries are placed above the lens, electrical connections can be made from above to below the lens with minimal vignetting if the wires are thin or of a transparent conductive material such as indium oxide.

Referring again to Figure 3a, Light-emitting diode (LED) illumination sources sit on a multilayer printed circuit board (PCB) outside the PAL and below the first refractive surface. LEDs are attractive sources due to their low cost, efficiency, compactness, and low coherence (which prevents speckle). Other illumination sources such as laser, incandescent, arc, or fluorescent sources are conceivable as well. Color photographs require a white light source. White LED's are available that may include a blue or violet LED along with phosphorescent materials that are excited by the LED and emit photons at longer wavelengths. A white light source may comprise a combination of LEDs or lasers emitting at different colors, such as red, green, and blue, within the visible spectrum and that together produce the same spectral response from the sensor (which has pixels sensitive to particular bands of color) as would a continuous-spectcum white light source.

The PCB that holds the LEDs may also serve as a mount for the LED and a baffle. and or aperture stop for the relay lens. Alternatively, strictures that perform these functions, along with the relay-lens barrel may attach to or support the PCB. LED drive circuits may be situated on the same PCB or on another PCB. Either way, control signals must be passed to the LEDs. Thus, some form of electrical connection between the LED-PCB and another PCB, for example the PCB that holds the sensor, is required. This connector might be a flexible PCB, a series of conductive pins or wires, or some other form of connector.

A potential problem with a capsule camera is that illumination light reflected by the exterior and interior surfaces of the capsule body may impinge on the sensor. Antireflection coatings can reduce these reflections, although adding these coatings to the capsule adds cost to the system. The correct geometry, however, ensures that the reflections will not overlap with the image on the sensor. US 6,836,377 and US6,918,872 disclose two such geometries for non-panoramic capsule cameras. In the first, the capsule dome is ellipsoidal with the sensor at its center and the LEDs lying on the focal curve. In the second, the dome is spherical with the sensor at the middle and the LEDs in the same plane further toward the edge of the sphere.

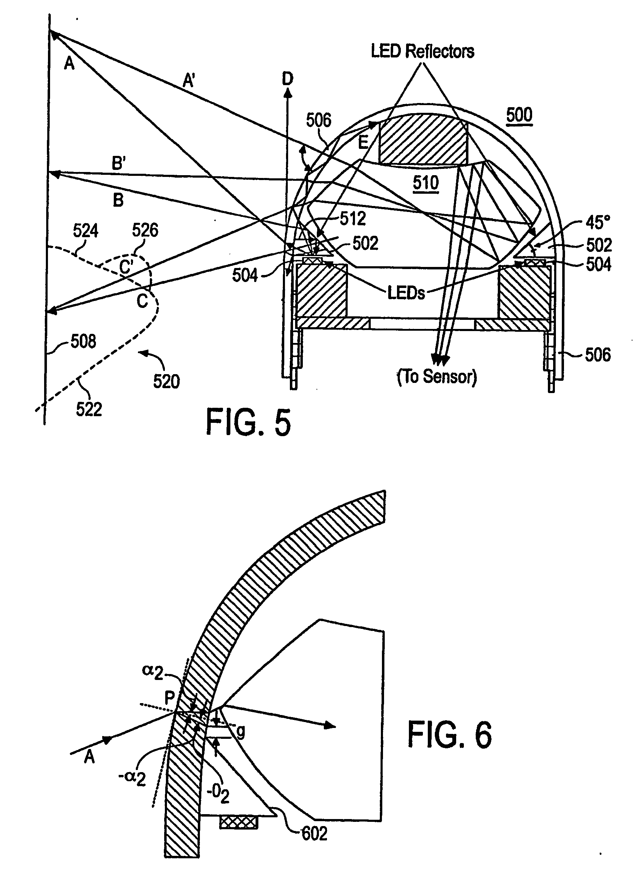

Figure 5 illustrates a geometry for a panoramic capsule camera where the LEDs lie below and to the outside of the first reflector. A reflective prism 502 sits above each LED 504. with one face of the prism at an angle roughly 45° relative to the longitudinal axis. This face may be coated with à metal or dielectric reflective coating that reflects light from the LED to the object or subject surface. In one embodiment, rather than use a separate prism for each LED, a continuous ring of triangular cross section may be formed into a single reflector 502. In preferred embodiment, the ring is made of a material whose refractive index:is nearly the same as that of the capsule housing506, and an index matching adhesive may be used to bond it to the housing such that reflections at the interface between the ring and the housing are minimize The figure shows rays A, B, and C passing from the LED 504 to the intestinal wall 508. Scattered light travels along paths A', B', and C' and is collected by lens 510 and imaged on the sensor (not shown). Reflections 512 will occur at the outer surface of the capsule. However, these reflections, shown as dotted lines, reflect back into the prism (or ring) and cannot be collected by the lens.

Still referring to Figure 5, two additional rays D and E are also shown. Ray D is light from reflector 502 and is refracted at an acute angle and would illuminate the intestinal wall within the field of view if the wall were closer. The reflection of ray D also passes back into the ring. Ray E clears the top of the ring. However, its angle of incidence upon the outer wall of the housing exceeds the critical angle for total internal reflection. The wall of the capsule serves as a light pipe that contains reflected light where much of it will scatter off the block positioned between the second reflector and the top of the capsule and away from the lends.

Also shown in Figure 5 is a ridge 520. As discussed in the background, such a ridge causes problems in viewing when using conventional devices, where prior art capsules can view and observe front side 522 as the capsule approaches the ridge, but misses the backside 524 because of a limited FOV. Polyp 526 or the area behind the polyp would normally be unobservable by a conventional device. In contrast, the invention provides a more useful and accurate means for observing the backside 524 and polyp 526, where the camera is able to view these surfaces, such as by ray C as illustrated. Ray C is then reflected as Ray C' that returns to the camera through the capsule shell and into lens 510. Thus, the invention provides a very useful and effective means to better observe these remote surfaces that were formerly not visible by prior art capsule cameras.

In Figure 6, ray A enters the capsule at point P at the largest field angle that the camera's aperture will capture. Within the body of the housing the ray makes an angle $\alpha_2$ with respect to the surface normal. If no rays can pass from the LED to point P at an, angle less than or equal to $\alpha_2$. then no reflected LED light from the housing outer wall will enter the camera aperture. As long as the distance g between the apex of the prism and the intersection of a line segment between point P, at an angle $-\alpha_2$ with the surface normal, and the inner wall of the housing, then this condition is satisfied, regardless of the exact position of the LED below the prism.

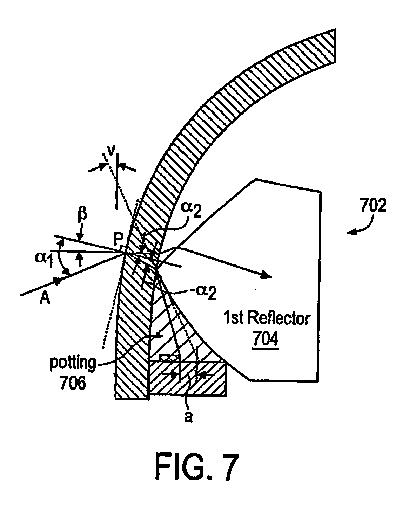

Figure 7 illustrates a similar embodiment of panoramic capsule camera 702 and LED illuminators 704. In this case, the region between the LED, the housing, and the outside of the first reflector is filled with an index-matching potting material 706. This region serves essentially the same illuminator function as the prism or ring 602 in the Figure 6. However, this approach eliminates the gap g of Figure 6. Ray A enters the capsule at point P at the largest field angle that is captured by the camera's aperture. The ray makes an angle $\alpha_1$ with the housing surface normal. The surface normal is at an angle $\beta$ with respect to the horizontal. Ray. A makes an angle $\alpha_2$ with respect to the surface normal within the housing wall where $n \sin \alpha_2 = \sin \alpha_1$ and n is the housing index of refraction. A ray emitted by the LED and reflects from the far end of the 1st reflector, passes into the housing and intersects the outer wall at point P. If this ray makes an angle of $-\alpha_2$ with the surface normal, then its reflection from the wall will be within the camera FOV. To ensure that no such rays exist, the LED must be placed a distance a or greater. away from the intersection of a line tangent to the first reflector at its outermost edge and the plane of the LED emitting surface. One can show that

$$a = h\left[\cot(\gamma) - \cot(2\gamma - \alpha_2 - \beta)\right]$$

where h is the distance from the LED plane to the ray-reflector intercept and $\gamma$ is the angle the tangent line makes with the LED plane.

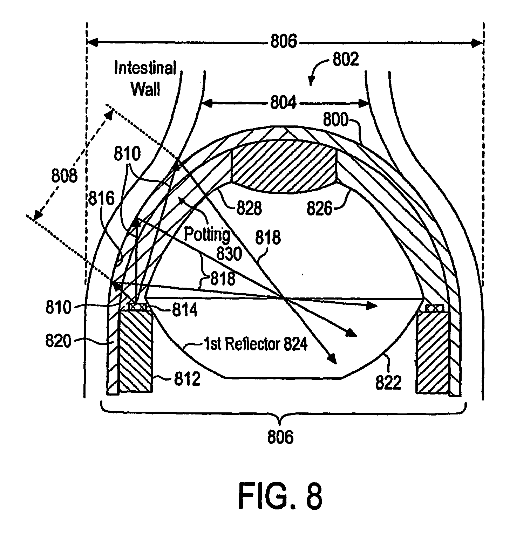

Referring to Figure 8, a panoramic capsule camera 800 is illustrated for imaging the small intestine or other organ having a cavity 802 that has a diameter 804 that is of comparable or smaller diameter to that of the capsule diameter 806. If comparable in diameter, then the capsule would fit close to the walls of the cavity. Thus, the cavity wall touches or is close to the outer wall of the capsule. If the diameter of the cavity is smaller that that of the capsule, the capsule would cause the inside surface of the cavity to expand around and against the capsule as it passes through the cavity, In the embodiment illustrated, the input pupil is the area 808 where light rays 810 are produced from the LED source 812 at output 814 and are reflected from the inner wall 816 of the cavity to enter back into the capsule as reflected rays 818. In this embodiment, the pupil 808 of the panoramic camera 808 is positioned at or near the center of curvature of the hemispherical or similarly shaped transparent capsule end dome 820. The LED output 814 is positioned such that light rays 810 emitted from the LED output 814 do not enter back into the camera in a manner that would reflect the LED rays 810 onto the first reflectors 822,824; then onto the surfaces 826,828 of second deflectors respectively and onto a sensor (not shown in Figure 8). Thus, any LED light reflected from either the inner or outer surfaces of the capsule are not collected by the camera input pupil and are not transmitted to the sensor to distort or otherwise affect the quality of the captured image. Optionally, index-matching potting adhesive 830 may be placed between the camera lenses reflective surfaces 828,826 and the capsule housing 820. Or, the lenses themselves may fit snuggly against the capsule housing with little or no gap between them. Still referring to Figure 8, the embodiment shows light LED rays 810 scattered by and reflected from the surface of the intestinal wall from various illuminating rays passing through the input pupil 808.

The invention may be implemented, for example, by having the image processing functions configured as a software application (as an operating system element), a dedicated processor, or a dedicated processor with dedicated code. There may be a small dedicated processor located within the capsule to perform limited functions in conjunction with a sensor to capture and transmit images. In one embodiment, the processor may be a simple logic circuit, dedicated microprocessor, integrated circuit, or other such processor configured to simply transmit image signals to a remote location, such as a proximally located receiver configured to receive the image signals embodying image data. From there, the image data can be stored, processed, forwarded, uploaded to another processor, or otherwise used.

The image data may be first compressed within the capsule, where a smaller amount of data can be transmitted to a remote location. This would reduce the power needed to transmit the information. The invention is directed to capturing a panoramic image by the capsule. Thus, the amount of information transmitted may be substantial, and compression of such data may be desired to reduce the amount transmitted. Transmission requires a good deal of power, and power conservation is very important. Thus, reducing the amount of transmitted date by compression prior to transmission may be desired. Many compression techniques are well known to those skilled in the art, and the invention is not limited to any particular method or technique.

The software executes a sequence of machine-readable instructions, which can also be referred to as code. These instructions may reside in various types of signal-bearing media. In this respect, one aspect of the present invention concerns a program product, comprising a, signal-bearing medium or signal-bearing media tangibly embodying a program of machine-readable instructions executable by a digital processing apparatus to perform methods of processing and other functions related to the invention described above.

This signal-bearing medium may comprise, for example, memory located in the capsule, memory in a host computer or in a server, depending on the particular configuration. The memory may be non-volatile storage, a data disc, or even memory on a dedicated server or vendor server for downloading to a processor for installation. Alternatively, the instructions may be embodied in a signal-bearing medium such as the optical data storage disc. Alternatively, the instructions may be stored on any of a variety of machine-readable data storage mediums or media; which may include, for example, a "hard drive", a RAID array, a RAMAC, a magnetic data storage diskette (such as a floppy disk), magnetic tape, digital optical tape, RAM, ROM, EPROM, EEPROM, flash memory, magneto-optical storage, paper.punch cards, or any other suitable signal-bearing media including transmission media such as digital and/or analog communications links, which may be electrical, optical, and/or wireless. As an example, the machine-readable instructions may comprise software object code, compiled from a language such as "C++".

Additionally, the program code may, for example, be compressed, encrypted, or both, and may include executable files, script files and wizards for installation, as in Zip files and cab files. As used herein the term machine-readable instructions or code residing in or on signal-bearing media include all of the above means of delivery.

While the foregoing disclosure shows a number of illustrative embodiments of the invention, it will be apparent to those skilled in the art that various changes and modifications can be made herein without departing from the scope of the invention as defined by the appended claims. Furthermore, although elements of the invention may be described or claimed in the singular, the plural is contemplated unless limitation to the singular is explicitly stated.

**Claims**

1. An in vivo image capturing system comprising:

   a capsule (500) enclosing a camera, the capsule comprising a transparent window configured to pass light into the camera;
   a light source (504) enclosed within the capsule;
   a prism or ring of triangular cross section (502, 602) configured within the capsule to reflect at least one ray of light (B) from the light source;
   whereby the transparent window comprises a point P on the outer surface, at which a ray A entering the capsule is captured at the largest field angle by the camera's aperture, and the transparent window is adapted to refract the ray A at an angle $\alpha_2$ with respect to the surface normal;
   wherein the prism or ring of triangular cross section is positioned at a distance g from the intersection of a line segment between point P, at an angle $-\alpha_2$ with the surface normal, and the inner wall of the transparent window to the apex of the prism or ring of triangular cross section; and
   such that wherever outgoing illuminating light rays after reflection by the prism or ring of triangular cross section intersect with incoming image rays (308) at the point P on the outer surface of the transparent window, an angle between an outgoing illuminating light ray reflected by the prism or ring of triangular cross section and a surface normal exceeds $|\alpha_2|$.

2. The in vivo image capturing system of claim 1, wherein the prism is configured to reflect a ray of light (B) from the illuminating light source, away from the camera, which is a panoramic camera, such that scattering of said ray (B) outside the capsule results in a scattered ray (B') entering the camera field of view.

3. The in vivo image capturing system of claim 1, wherein the camera is a panoramic camera including a lens (302) configured to receive illuminating light reflected from a subject surface, wherein the prism or ring of triangular cross section (502, 602) is configured to reflect illuminating light from the illuminating light source away from the panoramic camera in a manner that prevents stray light from directly entering the panoramic camera lens without being scattered by a subject surface.

4. The in vivo image capturing system of claim 1 wherein the camera is a panoramic camera having an entrance pupil (808), the camera is configured to capture a panoramic image of an environment surrounding the panoramic camera, and wherein the panoramic image is captured on a single image plane.

5. The in vivo image capturing system according to Claim 4, wherein the capsule comprises a closed housing including the transparent window for passing light into the panoramic camera, and wherein the panoramic camera is configured with a longitudinal field of view defined by a range of view angles relative to a longitudinal axis of the capsule and a latitudinal field of view defined by a panoramic range of azimuth angles about the longitudinal axis such that the panoramic camera can capture a panoramic image covering a 360 deg latitudinal FOV and a longitudinal FOV ranging from some angle greater than 0 deg to another angle greater than 90 deg.

6. The in vivo image capturing system according to Claim 5, wherein the panoramic camera includes a panoramic annular lens (302), and wherein the panoramic annular lens has a concentric axis of symmetry and includes two refractive surfaces (310, 322) and two reflective surfaces (314, 318) configured to allow incoming light to pass through a first refractive surface into a transparent medium.

7. The in vivo image capturing system according to Claim 4, wherein the panoramic camera includes a panoramic annular lens, wherein the panoramic annular lens has a concentric axis of symmetry and includes two refractive surfaces and two reflective surfaces configured to allow incoming light to pass through a first refractive surface into a transparent medium, to be reflected by a first reflective surface, then reflected by a second reflective surface, and then to exit the medium through a second refractive surface.

8. The in vivo image capturing system according to Claim 7, wherein the reflective and refractive surfaces are conicoid surfaces of revolution, and wherein the conic constant of the first reflective surface is in the range of -0.6 to +2, where the conic constant of the second reflective surface is chosen to be less than that of the first.

9. The in vivo image capturing system according to Claim 7, wherein the reflective and refractive surfaces are one of conicoid surfaces of revolution, spheroidal surfaces, or aspheroidal surfaces.

10. The in vivo image capturing system according to Claim 1, further comprising a data processor configured to generate a continuous image made up of multiple captured images.

11. The in vivo image capturing system according to Claim 10, wherein the data processor is located outside the capsule.

12. The in vivo image capturing system according to Claim 1, further comprising:

a power source for providing power to the light source and the camera, which is a panoramic camera; and
a memory configured to store captured images.

13. A method for in-vivo imaging with a panoramic camera with a field of view exceeding 180 degrees encased within a capsule, comprising:

providing a capsule (500) having a window at least partially transparent to electromagnetic radiation in a given wavelength range;
ingesting said capsule by a patient;
emitting electromagnetic radiation in said wavelength range from said capsule;
detecting images from reflections of said electromagnetic radiation with an image sensor using the panoramic camera;
wherein the capsule has inner and outer surfaces of the transparent window and
a prism or ring of triangular cross section (502, 602) configured to reflect a ray of light (B) from an illuminating light source, away from the panoramic camera;
whereby the transparent window comprises a point P on the outer surface, at which a ray A entering the capsule is captured at the largest field angle by the camera's aperture, and the transparent window refracts the ray A at an angle $\alpha_2$ with respect to the surface normal;
whereby the prism or ring of triangular cross section is positioned at a distance g from the intersection of a line segment between point P, at an angle $-\alpha_2$ with the surface normal, and the inner wall of the transparent window to the apex of the prism or ring of triangular cross section; and
such that wherever outgoing illuminating light rays (308) after reflection by the prism or ring of triangular cross section intersect with incoming image rays at the point P on the outer surface of the transparent window, an angle between an outgoing illuminating light ray reflected by the prism or ring of triangular cross section and a

surface normal exceeds $|\alpha_2|$,

14. A method according to Claim 13, further comprising performing compression on images detected by the image sensor to produce compressed image data; and uploading the compressed image data to a host computer.

15. The method according to Claim 14, further comprising after said performing and before said uploading, storing the compressed image data in a memory in the capsule.

16. The method according to claim 13, further comprising:

uploading the compressed image data to a computer for processing.

17. The method according to Claim 16, wherein:

uploading the images to a host computer further comprises retrieving the capsule housing and connecting the capsule to the computer.

18. The method according to Claim 16, wherein:

uploading the images to a computer further comprises transmitting the images from the capsule to the computer.

19. The method according to Claim 13, further comprising uploading the images with a wireless signal.

20. The method according to 13, further comprising performing compression on images resulting from said detecting, to produce compressed image data; and uploading the compressed image data via a wireless communication signal.

21. The method according to 13, further comprising performing compression on images detected by an image sensor in the capsule to produce compressed image data; storing the compressed image data in a memory in the capsule; and uploading the compressed image data via a wireless communication signal.

22. The method according to claim 13 wherein the panoramic camera field of view exceeds 180 degrees.

23. The method according to claim 13 further comprising generating a continuous image made up of multiple captured images.

24. The method according to claim 13, further comprising:

transmitting images captured by the panoramic camera to a remote receiver configured to receive captured images.

25. The method according to any one of claims 13 to 24, wherein the prism or ring of triangular cross section is configured to reflect a ray of light (B) from the illuminating light source, away from the panoramic camera such that scattering of said ray (B) outside the capsule results in a scattered ray (B') entering the camera field of view.

**Patentansprüche**

1. In-vivo-Bilderfassungssystem, umfassend:

eine Kapsel (500), die eine Kamera umschließt, wobei die Kapsel ein transparentes Fenster umfasst, das dazu eingerichtet ist, dass Licht in die Kamera passiert;
eine Lichtquelle (504), die in der Kapsel umschlossen ist;
ein Prisma oder ein Ring mit einem dreieckigen Querschnitt (502, 602), die innerhalb der Kapsel dazu eingerichtet sind, wenigstens einen Lichtstrahl (B) von der Lichtquelle zu reflektieren;
wobei das transparente Fenster einen Punkt P an der äußeren Oberfläche umfasst, an dem ein Strahl A, der in die Kapsel eintritt, mit dem größten Feldwinkel durch die Apertur der Kamera erfasst wird, und das transparente Fenster dazu ausgelegt ist, den Strahl A mit einem Winkel $\alpha_2$ in Bezug auf die Flächennormale zu brechen;
wobei das Prisma oder der Ring mit einem dreieckigen Querschnitt an einer Distanz g von dem Schnittpunkt eines Liniensegments zwischen Punkt P, bei einem Winkel von -$\alpha_2$ mit der Flächennormale, und der inneren Wand des transparenten Fensters zu dem Apex des Prismas oder des Rings mit einem dreieckigen Querschnitt angeordnet ist; und
so dass wo immer ausgehende Beleuchtungslichtstrahlen nach der Reflektion durch das Prisma oder den Ring mit einem dreieckigen Querschnitt sich mit eingehenden Lichtstrahlen (308) an dem Punkt P an der äußeren Oberfläche des transparenten Fensters schneiden, ein Winkel zwischen einem ausgehenden durch das Prisma oder den Ring mit einem dreieckigen Querschnitt reflektierten Beleuchtungslichtstrahl und einer Flächennormale $|\alpha_2|$ übersteigt.

2. In-vivo-Bilderfassungssystem nach Anspruch 1, wobei das Prisma dazu eingerichtet ist, einen Lichtstrahl (B) von der Beleuchtungslichtquelle weg von der Kamera, die eine Panoramakamera ist, so zu reflektieren, dass die Streuung des Strahls (B) außerhalb der Kapsel in einem gestreuten Lichtstrahl (B') resultiert, der in das Sichtfeld der Kamera eintritt.

3. In-vivo-Bilderfassungssystem nach Anspruch 1, wobei die Kamera eine Panoramakamera beinhaltend eine Linse (302) ist, die dazu eingerichtet ist, von einer Subjektsoberfläche reflektiertes Beleuchtungslicht zu empfangen, wobei das Prisma oder der Ring mit einem dreieckigen Querschnitt (502, 602) dazu eingerichtet sind, Beleuchtungslicht von der Beleuchtungslichtquelle von der Panoramakamera in einer Weise weg zu reflektieren, dass Streulicht daran gehindert ist, direkt in die Linse der Panoramakamera einzutreten, ohne durch eine Subjektsoberfläche gestreut zu sein.

4. In-vivo-Bilderfassungssystem nach Anspruch 1, wobei die Kamera eine Panoramakamera mit einer Eintrittspupille (808) ist, wobei die Kamera dazu eingerichtet ist, ein Panoramabild einer die Panoramakamera umgebenden Umgebung zu erfassen, und wobei das Panoramabild auf einer einzelnen Bildebene erfasst wird.

5. In-vivo-Bilderfassungssystem nach Anspruch 4, wobei die Kapsel ein geschlossenes Gehäuse beinhaltend das transparente Fenster zum Passieren von Licht in die Panoramakamera umfasst, und wobei die Panoramakamera mit einem longitudinalen Sichtfeld definiert durch einen Bereich von Sichtwinkeln relativ zu einer longitudinalen Achse der Kapsel und einem latitudinalen Sichtfeld definiert durch einen Panoramabereich von Azimuthwinkeln um die longitudinale Achse eingerichtet ist, so dass die Panoramakamera ein Panoramabild, das einen 360 Grad latitudinalen FOV und einen longitudinalen FOV im Bereich von einem Winkel größer als 0 Grad zu einem anderen Winkel größer als 90 Grad abdeckt, erfassen kann.

6. In-vivo-Bilderfassungssystem nach Anspruch 5, wobei die Panoramakamera eine ringförmige Panoramalinse (302) beinhaltet, und wobei die ringförmige Panoramalinse eine konzentrische Symmetrieachse aufweist und zwei lichtbrechende Oberflächen (310, 322) und zwei reflektierende Oberflächen (314, 318) beinhaltet, die dazu eingerichtet sind, es eingehendem Licht zu erlauben, durch eine erste lichtbrechende Oberfläche in ein transparentes Medium zu passieren.

7. In-vivo-Bilderfassungssystem nach Anspruch 4, wobei die Panoramakamera eine ringförmige Panoramalinse beinhaltet, und wobei die ringförmige Panoramalinse eine konzentrische Symmetrieachse aufweist und zwei lichtbrechende Oberflächen und zwei reflektierende Oberflächen beinhaltet, die dazu eingerichtet sind, es eingehendem Licht zu erlauben, durch eine erste lichtbrechende Oberfläche in ein transparentes Medium zu passieren, durch eine erste reflektierende Oberfläche reflektiert, dann durch eine zweite reflektierende Oberfläche reflektiert zu werden, und dann das Medium durch eine zweite lichtbrechende Oberfläche zu verlassen.

8. In-vivo-Bilderfassungssystem nach Anspruch 7, wobei die reflektierenden und lichtbrechenden Oberflächen konoide Oberflächen von Umdrehungen sind, und wobei die konische Konstante der ersten reflektierenden Oberfläche im Bereich von -0.6 bis +2 ist, und wobei die konische Konstante der zweiten reflektierenden Oberfläche niedriger als die von der ersten ausgewählt ist.

9. In-vivo-Bilderfassungssystem nach Anspruch 7, wobei die reflektierenden und lichtbrechenden Oberflächen eine von konoiden Oberflächen von Umdrehungen, spheroidalen Oberflächen oder aspheroidalen Oberflächen sind.

10. In-vivo-Bilderfassungssystem nach Anspruch 1, ferner umfassend einen Datenprozessor, der dazu eingerichtet ist, ein kontinuierliches Bild aufgebaut aus mehreren erfassten Bilder zu erzeugen.

11. In-vivo-Bilderfassungssystem nach Anspruch 10, wobei der Datenprozessor außerhalb der Kapsel angeordnet ist.

12. In-vivo-Bilderfassungssystem nach Anspruch 1, ferner umfassend:

    eine Energiequelle zum Bereitstellen von Energie für die Lichtquelle und die Kamera, welche eine Panoramakamera ist; und
    einen Speicher, der dazu eingerichtet ist, erfasste Bilder zu speichern.

13. Verfahren zum in-vivo-Abbilden mit einer Panoramakamera mit einem 180 Grad überschreitenden Sichtfeld, die innerhalb einer Kapsel eingeschlossen ist, umfassend:

    Bereitstellen einer Kapsel (500) mit einem Fenster, das wenigstens teilweise für elektromagnetische Strahlung in einem gegebenen Wellenlängenbereich transparent ist;
    Aufnehmen der Kapsel durch einen Patienten;
    Emittieren von elektromagnetischer Strahlung in dem Wellenlängenbereich von der Kapsel;
    Detektieren von Bildern von Reflektionen von der elektromagnetischen Strahlung mit einem Bildsensor unter Verwendung der Panoramakamera;

wobei die Kapsel innere und äußere Oberflächen des transparenten Fensters und ein Prisma oder einen Ring mit dreieckigen Querschnitt (502, 602) aufweist, die dazu eingerichtet sind, einen Lichtstrahl (B) von einer Beleuchtungslichtquelle weg von der Panoramakamera zu reflektieren,

wobei das transparente Fenster einen Punkt P an der äußeren Oberfläche umfasst, an dem ein Strahl A, der in die Kapsel eintritt, mit dem größten Feldwinkel durch die Apertur der Kamera erfasst wird, und das transparente Fenster den Strahl A mit einem Winkel $\alpha_2$ in Bezug auf die Flächennormale bricht;

wobei das Prisma oder der Ring mit einem dreieckigen Querschnitt an einer Distanz g von dem Schnittpunkt eines Liniensegments zwischen Punkt P, bei einem Winkel von -$\alpha_2$ mit der Flächennormale, und der inneren Wand des transparenten Fensters zu dem Apex des Prismas oder des Rings mit einem dreieckigen Querschnitt angeordnet ist; und

so dass wo immer ausgehende Beleuchtungslichtstrahlen (308) nach der Reflektion durch das Prisma oder den Ring mit einem dreieckigen Querschnitt sich mit eingehenden Lichtstrahlen an dem Punkt P an der äußeren Oberfläche des transparenten Fensters schneiden, ein Winkel zwischen einem ausgehenden durch das Prisma oder den Ring mit einem dreieckigen Querschnitt reflektierten Beleuchtungslichtstrahl und einer Flächennormale $|\alpha_2|$ übersteigt.

14. Verfahren nach Anspruch 13, ferner umfassend Durchführen einer Komprimierung der durch den Bildsensor detektierten Bilder, um komprimierte Bilddaten herzustellen; und Heraufladen der komprimierten Bilddaten zu einem Hostcomputer.

15. Verfahren nach Anspruch 14, ferner umfassend, nach dem Durchführen und vor dem Heraufladen, Speichern der komprimierten Bilddaten in einem Speicher in der Kapsel.

16. Verfahren nach Anspruch 13, ferner umfassend:

Heraufladen der komprimierten Bilddaten zu einem Computer zum Bearbeiten.

17. Verfahren nach Anspruch 16, wobei:

das Heraufladen der Bilder zu einem Hostcomputer ferner das Wiedergewinnen des Kapselgehäuses und das Verbinden der Kapsel zu dem Computer umfasst.

18. Verfahren nach Anspruch 16, wobei:

das Heraufladen der Bilder zu einem Computer ferner das Senden der Bilder von der Kapsel zu dem Computer umfasst.

19. Verfahren nach Anspruch 13, ferner umfassend Heraufladen der Bilder mit einem drahtlosen Signal.

20. Verfahren nach Anspruch 13, ferner umfassend Durchführen einer Komprimierung von aus dem Detektieren resultierenden Bildern, um komprimierte Bilddaten herzustellen; und Heraufladen der komprimierten Bilddaten über ein drahtloses Kommunikationssignal.

21. Verfahren nach Anspruch 13, ferner umfassend Durchführen einer Komprimierung von Bildern, die durch einen Bildsensor in der Kapsel detektiert sind, um komprimierte Bilddaten herzustellen; Speichern der komprimierten Bilddaten in einem Speicher in der Kapsel; und Heraufladen der komprimierten Bilddaten über ein drahtloses Kommunikationssignal.

22. Verfahren nach Anspruch 13, wobei das Sichtfeld der Panoramakamera 180 Grad überschreitet

23. Verfahren nach Anspruch 13, ferner umfassend Erzeugen eines kontinuierlichen Bildes, das aus mehreren erfassten Bildern zusammengesetzt ist.

24. Verfahren nach Anspruch 13, ferner umfassend:

Senden von durch die Panoramakamera erfassten Bildern zu einem entfernten Empfänger, der dazu eingerichtet ist, erfasste Bilder zu empfangen.

25. Verfahren nach einem der Ansprüche 13 bis 24, wobei das Prisma oder der Ring mit einem dreieckigen Querschnitt dazu eingerichtet sind, einen Lichtstrahl (B) von einer Beleuchtungslichtquelle weg von der Panoramakamera so zu reflektieren, dass die Streuung des Strahls (B) außerhalb der Kapsel in einem gestreuten Lichtstrahl (B') resultiert, der in das Sichtfeld der Kamera eintritt.

**Revendications**

1. Système de capture d'images in vivo comprenant :

une capsule (500) contenant une caméra, la capsule comprenant une fenêtre transparente configurée pour faire passer la lumière dans la caméra ;
une source lumineuse (504) contenue dans la capsule ;

un prisme ou anneau de section transversale triangulaire (502, 602) configuré à l'intérieur de la capsule pour réfléchir au moins un rayon lumineux (B) provenant de la source lumineuse ; la fenêtre transparente comprenant un point P sur la surface externe, au niveau duquel un rayon A entrant dans la capsule est capturé à l'angle de champ le plus grand par l'ouverture de la caméra, et la fenêtre transparente est adaptée pour réfracter le rayon A selon un angle $\alpha_2$ par rapport à la surface perpendiculaire ;

dans lequel le prisme ou anneau de section transversal triangulaire est positionné à une distance g de l'intersection d'un segment de ligne entre un point P, à un angle - $\alpha_2$ avec la surface perpendiculaire, et de la paroi interne de la fenêtre transparente au sommet du prisme ou anneau de section transversale triangulaire ; et

de telle sorte que quel que soit l'endroit où des rayons lumineux éclairant de sortie, après réflexion par le prisme ou anneau de section transversale triangulaire, s'entrecoupent avec des rayons d'image entrant (308) au point P sur la surface externe de la fenêtre transparente, un angle entre un rayon lumineux éclairant de sortie réfléchi par le prisme ou anneau de section transversale triangulaire et une surface perpendiculaire est supérieur à |$\alpha_2$|.

2.  Système de capture d'images in vivo selon la revendication 1, dans lequel le prisme est configuré pour réfléchir un rayon de lumière (B) provenant de la source lumineuse éclairante, en éloignement de la caméra, qui est une caméra panoramique, de sorte que la diffusion dudit rayon (B) à l'extérieur de la capsule résulte en un rayon diffusé (B') entrant dans le champ de vision de la caméra.

3.  Système de capture d'images in vivo selon la revendication 1, dans lequel la caméra est une caméra panoramique comprenant une lentille (302) configurée pour recevoir une lumière éclairante réfléchie par une surface objet, dans lequel le prisme ou anneau de section transversale triangulaire (502, 602) est configuré pour réfléchir une lumière éclairante provenant de la source lumineuse éclairante en éloignement de la caméra panoramique d'une manière qui empêche la lumière parasite d'entrer directement dans la lentille de la caméra panoramique sans être diffusée par une surface objet.

4.  Système de capture d'images in vivo selon la revendication 1, dans lequel la caméra est une caméra panoramique ayant une pupille d'entrée (808), la caméra est configurée pour capturer une image panoramique d'un environnement entourant la caméra panoramique, et dans lequel l'image panoramique est capturée sur un seul plan d'image.

5.  Système de capture d'images in vivo selon la revendication 4, dans lequel la capsule comprend un logement fermé comprenant la fenêtre transparente pour faire passer une lumière dans la caméra panoramique, et dans lequel la caméra panoramique est configurée avec un champ de vision longitudinal défini par une série d'angles de vision par rapport à un axe longitudinal de la capsule et un champ de vision latitudinal défini par une zone panoramique d'angles d'azimut autour de l'axe longitudinal de telle sorte que la caméra panoramique peut capturer une image panoramique couvrant un champ de vision latitudinal à 360° et un champ de vision longitudinal allant d'un certain angle supérieur à 0 degré à un autre angle supérieur à 90 degrés.

6.  Système de capture d'images in vivo selon la revendication 5, dans lequel la caméra panoramique comprend une lentille annulaire panoramique (302), et dans lequel la lentille annulaire panoramique a un axe de symétrie concentrique et comprend deux surfaces de réfraction (310, 322) et deux surfaces de réflexion (314, 318) configurées pour permettre à la lumière entrante de passer à travers une première surface de réfraction dans un milieu transparent.

7.  Système de capture d'images in vivo selon la revendication 4, dans lequel la caméra panoramique comprend une lentille annulaire panoramique, dans lequel la lentille annulaire panoramique a un axe de symétrie concentrique et comprend deux surfaces de réfraction et deux surfaces de réflexion configurées pour permettre à la lumière entrante de traverser une première surface de réfraction dans un milieu transparent, pour être réfléchie par une première surface de réflexion, ensuite réfléchie par une deuxième surface de réflexion, et ensuite pour sortir du milieu au travers d'une deuxième surface de réfraction.

8.  Système de capture d'images in vivo selon la revendication 7, dans lequel les surfaces de réflexion et de réfraction sont des surfaces de révolution conicoides, et dans lequel la constante conique de la première surface de réflexion est située dans la plage de -0,6 à +2, où la constante conique de la deuxième surface de réflexion est choisie pour être inférieure à celle de la première.

9.  Système de capture d'images in vivo selon la revendication 7, dans lequel les surfaces de réflexion et de réfraction sont une des surfaces de révolution conicoides, des surfaces sphéroïdales, ou des surfaces asphéroïdales.

10. Système de capture d'images in vivo selon la revendication 1, comprenant en outre un processeur de données configuré pour générer une image continue composée de multiples images capturées.

11. Système de capture d'images in vivo selon la revendication 10, dans lequel le processeur de données est situé à l'extérieur de la capsule.

12. Système de capture d'images in vivo selon la revendication 1, comprenant en outre :

une source d'énergie pour fournir de l'énergie à la source lumineuse et à la caméra qui est une caméra panoramique ; et
une mémoire configurée pour stocker des images capturées.

13. Procédé d'imagerie in vivo avec une caméra panoramique avec un champ de vision dépassant 180 degrés emboîté à l'intérieur d'une capsule, comprenant :

la fourniture d'une capsule (500) ayant une fenêtre au moins partiellement transparente à une radiation électromagnétique dans une gamme de longueurs d'ondes donnée ;
l'ingestion de ladite capsule par un patient ;
l'émission d'une radiation électromagnétique dans ladite gamme de longueurs d'onde à partir de ladite capsule ;
la détection d'images à partir des réflexions de ladite radiation électromagnétique avec un capteur d'images utilisant la caméra panoramique ;
dans lequel la capsule a des surfaces interne et externe de la fenêtre transparente et un prisme ou anneau de section transversale triangulaire (502, 602) configuré pour réfléchir un rayon de lumière (B) provenant d'une source lumineuse éclairante, en éloignement de la caméra panoramique ;
la fenêtre transparente comprenant un point P sur la surface externe, au niveau duquel un rayon A entrant dans la capsule est capturé à l'angle de champ le plus grand par l'ouverture de la caméra, et la fenêtre transparente réfracte le rayon A selon un angle $\alpha_2$ par rapport à la surface perpendiculaire ;
dans lequel le prisme ou anneau de section transversal triangulaire est positionné à une distance g de l'intersection d'un segment de ligne entre un point P, à un angle - $\alpha_2$ avec la surface perpendiculaire, et de la paroi interne de la fenêtre transparente au sommet du prisme ou anneau de section transversale triangulaire ; et
de telle sorte que quel que soit l'endroit où des rayons lumineux éclairant de sortie (308), après réflexion par le prisme ou anneau de section transversale triangulaire, s'entrecoupent avec des rayons d'image entrant au point P sur la surface externe de la fenêtre transparente, un angle entre un rayon lumineux éclairant de sortie réfléchi par le prisme ou anneau de section transversale triangulaire et une surface perpendiculaire dépasse $|\alpha_2|$,

14. Procédé selon la revendication 13, comprenant en outre
la réalisation d'une compression sur les images détectées par le capteur d'images pour produire des données d'image compressées ; et
le téléchargement des données d'image compressées vers un ordinateur hôte.

15. Procédé selon la revendication 14, comprenant en outre
après ladite réalisation et avant ledit téléchargement, le stockage des données d'image compressées dans une mémoire dans la capsule.

16. Procédé selon la revendication 13, comprenant en outre :

le téléchargement des données d'image compressées vers un ordinateur pour traitement.

17. Procédé selon la revendication 16, dans lequel :

le téléchargement des images vers un ordinateur hôte comprend en outre la récupération du boîtier de capsule et le raccordement de la capsule à l'ordinateur.

18. Procédé selon la revendication 16, dans lequel :

le téléchargement des images vers un ordinateur comprend en outre la transmission des images depuis la capsule vers l'ordinateur.

19. Procédé selon la revendication 13, comprenant en outre le téléchargement des images avec un signal sans fil.

20. Procédé selon la revendication 13, comprenant en outre
la réalisation de compression sur des images résultant de ladite détection, pour produire des données d'image compressées ; et
le téléchargement des données d'image compressées par l'intermédiaire d'un signal de communication sans fil.

21. Procédé selon la revendication 13, comprenant en outre
la réalisation d'une compression sur des images détectées par un capteur d'images dans la capsule pour produire des données d'image compressées ;

le stockage des données d'image compressées dans une mémoire dans la capsule ; et le téléchargement des données d'image compressées par l'intermédiaire d'un signal de communication sans fil.

**22.** Procédé selon la revendication 13, dans lequel le champ de vision de la caméra panoramique dépasse 180 degrés.

**23.** Procédé selon la revendication 13, comprenant en outre
la génération d'une image continue composée de multiples images capturées.

**24.** Procédé selon la revendication 13, comprenant en outre :

la transmission d'images capturées par la caméra panoramique vers un récepteur à distance configuré pour recevoir des images capturées.

**25.** Procédé selon l'une quelconque des revendications 13 à 24, dans lequel le prisme ou anneau de section transversale triangulaire est configuré pour réfléchir un rayon lumineux (B) depuis la source lumineuse éclairante, en éloignement de la caméra panoramique de telle sorte que la diffusion dudit rayon (B) à l'extérieur de la capsule résulte en un rayon diffusé (B') entrant dans le champ de vision de la caméra.

FIG. 1

EP 1 974 240 B1

FIG. 2

18

Second Reflector 318

308

First Refractor 310

308

304
305

308

Second
Refractor 322

Relay Lens
System 327

Electrical
Connector 346

312    320

324

326          328

330

332
334
336

338

Sensor 340

PCB 342

302
316

First Reflector 314
LED Reflectors 306
LEDs 304
PCB 305
307

300

344

FIG. 3A

FIG. 3B

EP 1 974 240 B1

FIG. 4

LED Reflectors

A

A'

D

506

E

500

510

B'

512

B

45°

524    526

502

502

C'

504

504

664

C

LEDs

508

(To Sensor)

506

520

522

**FIG. 5**

α2

P

g

A

−α2

−θ2

602

**FIG. 6**

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 20050146644 A
- WO 2004096008 A2
- WO 9502841 A1
- US 20030171653 A1
- US 4566763 A
- US 5473474 A
- US 6836377 B
- US 6918872 B

**Non-patent literature cited in the description**

- Characterization of the panoramic annular lens. **LEHNER, D. L et al.** Experimental Mechanics. Springer, 01 December 1996, vol. 36, 333-338